# EUROPEAN PATENT APPLICATION

(11) **EP 4 596 020 A1**
(43) Date of publication of application: **06.08.2025**
(21) Application number: 23872805.9
(22) Date of filing: 18.08.2023
(51) Int. Cl.: A61M 37/00, B29C 33/38, B29C 37/00, B29C 45/00, B29C 43/20, B29C 33/42, A61F 13/02

(54) **SKIN ADHESIVE PATCH FOR NEGATIVE PRESSURE STIMULATION AND MANUFACTURING METHOD THEREFOR**

(30) Priority: 27.09.2022 KR 20220122792; 16.08.2023 KR 20230107150
(71) Applicant: Research & Business Foundation SungKyunKwan University, Gyeonggi-do 16419 (KR)
(72) Inventor: PANG, Chang Hyun, Suwon-si Gyeonggi-do 16419 (KR); LEE, Ji Hyun, Suwon-si Gyeonggi-do 16419 (KR); PARK, Hyoung Ki, Suwon-si Gyeonggi-do 16419 (KR)
(74) Representative: Patrade A/S
(86) International application number: PCT/KR2023/012224
(87) International publication number: WO 2024/071682

(57) **Abstract**

An embodiment of the present invention provides a skin adhesive patch for negative pressure stimulation and a manufacturing method thereof. The skin adhesive patch mimics an embossed octopus suction cup-inspired adhesive structure that can allows stable attachment and detachment even under dry or moist skin conditions. The skin adhesive patch is provided with an adhesive cup portion having a deformable curvature, which allows the suction cup to conform to the skin and cover a wider surface area upon attachment. Therefore, when adhering to the skin, the skin adhesive patch locally induces negative pressure on the skin, thereby increasing the delivery efficiency of active ingredients through the skin without an external power source.

## Description

### [TECHNICAL FIELD]

The present invention relates to a skin adhesive patch, and more particularly, to a negative pressure stimulation adhesive skin patch that locally induces negative pressure to improve transdermal delivery efficiency of active ingredients without requiring an external power source, as well as a method for manufacturing the same.

### [BACKGROUND ART]

Keeping pace with the rapid growth of the transdermal drug delivery system market, the development of microscale structure-based adhesive patch fabrication technology that adheres to the skin without causing irritation, along with systems capable of inducing localized negative pressure on the skin without an external power source, holds great potential for applications in medical wearable devices and drug delivery therapeutic patches.

With recent advancements in wearable drug delivery systems, transdermal drug delivery devices capable of adhering to the skin for drug administration have been receiving significant attention.

Transdermal drug delivery is a system that administers drugs into the body through skin absorption, making it useful for a wide range of drugs and medical conditions.

Transdermal drug delivery systems can reduce side effects compared to oral administration, enhance bioavailability by bypassing hepatic metabolism, and improve patient compliance and convenience compared to injection-based drug delivery.

However, for effective transdermal drug delivery, it is essential to overcome the mechanical barrier of the stratum corneum, which serves as the primary defense layer of the skin, and to enhance drug penetration and absorption rates.

To this end, commercially available drug delivery devices have been developed utilizing various technologies, including the induction of physical perforation in the stratum corneum through electrical stimulation, invasive drug delivery via microneedles, photothermal stimulation-assisted drug delivery, and the application of chemical enhancers to improve drug delivery efficiency.

One example of transdermal drug delivery technology involves inducing micro-perforations in the skin layer using strong magnetic pressure to enhance drug delivery efficiency. However, the high pressure required to create these perforations may lead to skin irritation. Additionally, achieving sufficient pressure necessitates prolonged attachment of the magnet, posing challenges for commercialization.

Another example of transdermal drug delivery technology involves inducing partial etching of the skin layer using near-infrared photothermal effects. This method has demonstrated higher drug delivery efficiency compared to the use of chemical enhancers. However, drug delivery through photothermal stimulation may cause secondary side effects, such as skin damage resulting from the removal of the stratum corneum.

Another example of transdermal drug delivery technology involves enhancing drug delivery efficiency by inducing pressure through a suction cup after drug injection. However, this method requires a vacuum pump to generate the necessary pressure, leading to increased complexity and inconvenience due to the additional equipment. Furthermore, it necessitates the prolonged application of negative pressure, which poses challenges for practical implementation and user convenience.

Another example of transdermal drug delivery technology is a patch-type transdermal drug delivery product. This type of product typically includes a drug layer and an adhesive layer, with most adhesive layers utilizing chemical adhesive materials. However, such chemical adhesives may leave residues on the skin or cause irritation.

That is, transdermal drug delivery technology still poses the following problems.

Despite advancements in transdermal drug delivery technology, several challenges remain.

First, when applying electrical stimulation, there is the problem of requiring an external power source. Second, there is a risk of secondary skin damage caused by electrical stimulation. Third, invasive methods using microneedles pose a risk of bacterial infection. Fourth, the use of chemical adhesives to ensure close contact between the drug delivery material and the skin interface may lead cause side effects such as skin irritation, peeling, and redness.

In other words, conventional technologies developed to enhance the efficiency of transdermal drug delivery are subject to technical and material limitations, including concerns regarding skin damage, the necessity of an external power source, and insufficient skin adhesion performance and stability.

Accordingly, there is a need to develop a technology capable of addressing the above-described problems associated with conventional transdermal drug delivery techniques.

### [PRIOR ART DOCUMENTS]

Korean Laid-Open Patent No. 10-2018-0065848 (Published on June 18, 2018)

### [DETAILED DESCRIPTION OF THE INVENTION]

### [TECHNICAL PROBLEM]

The present invention aims to address the aforementioned issues of conventional technologies. The present invention may provide a negative pressure stimulation adhesive skin patch and a method for manufacturing the same, which locally induces negative pressure to enhance the efficiency of transdermal delivery of active ingredients without the need for an external power source.

Furthermore, another objective of the present invention may be to provide a negative pressure stimulation adhesive skin patch and a method for manufacturing the same, the patch including an embossed octopus-inspired suction structure that enables stable attachment and detachment in both dry and moist skin environments, and including an adhesive cup portion having a deformable curvature, which allows the suction cup to conform to the skin and cover a wider surface area upon attachment.

Furthermore, another objective of the present invention may be to provide a negative pressure stimulation adhesive skin patch and a method for manufacturing the same, which are designed to minimizes skin irritation and side effects caused by transdermal drug delivery materials.

Still another objective of the present invention may be to provide a negative pressure stimulation adhesive skin patch and a method for manufacturing the same, wherein localized negative pressure is induced by the adhesive suction cups based on stable skin adhesion performance, thereby enhancing the efficiency of transdermal drug delivery.

The technical problems addressed by the present invention are not limited to those explicitly mentioned above. Other technical challenges that have not been specifically stated will be readily understood by those skilled in the art from the descriptions provided below.

### [MEANS FOR SOLVING THE PROBLEMS]

In order to achieve the aforementioned technical objectives, an embodiment of the present invention provides a negative pressure stimulation adhesive skin patch, including: a flexible base surface portion (10); and a plurality of adhesive cup portions (20) arranged on the skin-contacting surface of the base surface portion (10).

The base surface portion (10) and the adhesive cup portions (20) may be formed from one or more flexible polymer materials selected from the group consisting of polydimethylsiloxane (PDMS), Ecoflex, polyurethane (PU), polyaniline (PANI), and styrene-ethylene/butylene-styrene (SEBS).

The adhesive cup portion (20) may include: a cup column portion (21) having a negative pressure groove (22) formed on its skin-contacting surface; an adhesive flange (23) extending laterally from the upper rim region of the cup column portion (21); and a dome (25) protruding in relief from the bottom surface of the negative pressure groove (22).

The cup column portion (21) may be formed in a columnar shape with concave side surfaces, wherein the side generatrix may be formed as a concave arc along the vertical central axis of the cup column portion (210).

The adhesive cup portion (20) may further include a polymer precursor coating layer (50) formed on the upper surface of the adhesive flange portion (23) to smooth the surface roughness and enhance adhesive strength.

The polymer precursor forming the polymer precursor coating layer (50) may include at least one selected from the group consisting of PDMS (Polydimethylsiloxane), Ecoflex, PU (Polyurethane), PANI (Polyaniline), and SEBS (Styrene-Ethylene/Butylene-Styrene).

In order to achieve the above-described technical objectives, another embodiment of the present invention may include designing a 3D mold model having a recessed structure corresponding to a plurality of adhesive cup portions (20), for use in molding a skin-adhesive patch for applying negative pressure stimulation to the skin; fabricating a 3D mold (30) based on the designed 3D mold model; molding a skin adhesive patch (1) including a base surface portion (10) and a plurality of adhesive cup portions (20) by depositing a flexible polymer material onto a surface-treated 3D mold (30) and curing the material; releasing the molded skin-adhesive patch (1) from the 3D mold (30); and forming a polymer precursor coating layer (50) on a skin-adhesive surface of the skin-adhesive patch (1).

The 3D mold manufacturing may further include surface-treating the 3D mold.

The molding may be performed through a solution process using a flexible polymer material.

In the molding, the flexible polymer material may include at least one selected from the group consisting of PDMS (Polydimethylsiloxane), Ecoflex, PU (Polyurethane), PANI (Polyaniline), and SEBS (Styrene-Ethylene/Butylene-Styrene).

The polymer precursor coating layer forming may include a stamping process in which a polymer precursor is laminated on a skin-adhesive surface of the skin-adhesive patch (1), followed by performing a stamping procedure to form the polymer precursor coating layer (50) and an adhesive flange portion (23).

The polymer precursor coating layer forming may include a spray coating process in which a polymer precursor is sprayed onto a skin-adhesive surface of the skin-adhesive patch (1) to form the polymer precursor coating layer (50).

The polymer precursor coating layer forming may include spraying a polymer precursor onto a skin-adhesive surface of the skin-adhesive patch (1) to form the polymer precursor coating layer (50).

In the polymer precursor coating layer forming, the polymer precursor may include at least one selected from the group consisting of PDMS (Polydimethylsiloxane), Ecoflex, PU (Polyurethane), PANI (Polyaniline), and SEBS (Styrene-Ethylene/Butylene-Styrene).

### [EFFECTS OF THE INVENTION]

An embodiment of the present invention may provide effects of addressing issues such as the need for an external power source in drug delivery systems, the use of structures and materials that may cause secondary conditions such as skin infections, rashes, and redness, and insufficient adhesive performance for stable skin attachment.

Furthermore, an embodiment of the present invention provides the effect of enhancing the efficiency of active ingredient delivery through the skin without requiring an external power source by inducing localized negative pressure.

In addition, the above-described embodiment of the present invention may provide an effect of increasing the delivery efficiency of active ingredients through the skin without an external power source by locally inducing negative pressure.

In addition, an embodiment of the present invention may provide an effect of improving skin keratin softening and drug absorption capability by mimicking a protruded octopus suction cup-inspired adhesive structure that allows stable attachment and detachment even under dry or moist skin conditions, and by having a freely deformable curvature such that the adhesive cups can cover a broader area when adhered to the skin.

In addition, an embodiment of the present invention may provide an effect of enabling negative pressure stimulation that minimizes skin irritation or side effects caused by transdermal drug delivery materials. Furthermore, an embodiment of the present invention may provide an effect of serving as a foundational or original technology for high-performance medical patch systems such as drug delivery systems and skin-interface sensors, by being combinable with various delivery agents or electronic materials in conjunction with existing transdermal drug delivery technologies.

The effects of the present invention are not limited to those described above, and should be understood to include all effects that can be reasonably inferred from the configurations of the invention described in the detailed description or the claims.

### [BRIEF DESCRIPTION OF THE DRAWINGS]

FIG. 1 is a perspective view of a skin-adhesive patch (1) capable of providing negative pressure stimulation according to an embodiment of the present invention and an enlarged cross-sectional view of an adhesive cup portion (20).
FIG. 2 includes a photograph, a cross-sectional view, and a design drawing of a large-area patch showing the structure of a protruded octopus-inspired adhesive cup having microdome structures and curvature.
FIG. 3 is a schematic diagram illustrating manufacturing of an adhesive patch according to an embodiment of the present invention.
FIG. 4 is a graph showing vertical adhesion force measurement data according to the scale (diameter: 2, 3, 5, 10 mm) of the adhesive structure under dry and moist environmental conditions.
FIG. 5 illustrates (a) photographs showing the increasing contact area of the adhesive cup portion (20) on the skin contact surface during preload, and (b) a graph showing the coverage ratio, which indicates the area that the adhesive cup portion (20) is capable of covering, depending on the outer diameter of the skin-contact surface of the adhesive cup portion (20), including (i) 2 mm, (ii) 3 mm, (iii) 5 mm, and (iv) 10 mm.
FIG. 6 shows the adhesive performance of the patch on a pig skin replica, including: (a) a graph of vertical adhesion performance according to preload; (b) a graph comparing horizontal and peel adhesion performance between an octopus-inspired adhesive structure (d-SCC) and a flat patch without such a structure; and (c) a graph showing the adhesion performance of the d-SCC after 100 repeated attachment cycles.
FIG. 7 includes: (a) schematic diagrams showing the cases where the d-SCC is applied and not applied; (b) graphs presenting penetration depth data of fluorescent particles (rhodamine B) delivered into pig skin depending on the application time (5, 10, 20, and 30 minutes) of the d-SCC; and (c) photographs showing the distribution of fluorescent particles (rhodamine B) in the skin according to the application time (5, 10, 20, and 30 minutes) of the d-SCC.
FIG. 8 includes: (a) a schematic diagram illustrating the state after removal of the d-SCC; (b) graphs showing penetration depth data of fluorescent particles (rhodamine B) into pig skin at different time intervals (5, 10, 20, and 30 minutes) after removal of the d-SCC that had been applied for 30 minutes; and (c) photographs showing the distribution of fluorescent particles (rhodamine B) in the skin at 5, 10, 20, and 30 minutes after removal of the d-SCC following 30 minutes of application.
FIG. 9 is a graph showing the measured penetration depth of fluorescent particles (rhodamine B) enhanced by the d-SCC across various skin types, including pig skin, human skin, and artificial skin.
FIG. 10 is an image showing deformation of the stratum corneum of pig skin according to preload.
FIG. 11 includes: (a) a graph showing the enhancement in penetration depth by the d-SCC for various drugs with different molecular weights applied to pig skin; and (b) photographs showing the distribution of various drugs delivered under each condition.
FIG. 12 includes: (a) photographs showing the application of a prototype product (surgical plastic tape and chlorhexidine gluconate film) and the d-SCC patch according to an embodiment of the present invention to various human skin types; and (b) a graph comparing skin irritation levels caused by the prototype products and the d-SCC patch of the present invention.

RIG. 13 includes: (a) photographs showing the skin recovery progress in mice with atopic dermatitis treated with various drugs in combination with the d-SCC; and (b) a graph showing transepidermal water loss (TEWL) over time when various drugs are applied in combination with the d-SCC.

### [MODE FOR CARRYING OUT THE INVENTION]

Hereinafter, the present invention will be described with reference to the accompanying drawings. However, the present invention is not limited to the embodiments described herein and may be implemented in various other forms. Parts that are not related to the description are omitted from the drawings to clearly illustrate the present invention, and similar reference numerals are used for similar elements throughout the specification.

In the present specification, when a part is described as being "connected," "coupled," or "in contact" with another part, this includes not only cases where the parts are directly connected, coupled, or in contact, but also cases where they are indirectly connected, coupled, or in contact via one or more other elements.

Furthermore, when a part is described as "including" a certain component, unless explicitly stated otherwise, it is to be understood that the part may further include other components in addition to the stated one, and that the inclusion does not exclude the presence of other elements.

The terminology used herein is intended to describe particular embodiments only and is not intended to limit the scope of the present invention. As used in the specification, the singular forms also include the plural forms unless the context clearly dictates otherwise.

The terms "include," "have," and their variations are used to specify the presence of stated features, numbers, steps, operations, components, or combinations thereof, but do not preclude the presence or addition of one or more other features, numbers, steps, operations, components, or combinations thereof.

Hereinafter, exemplary embodiments of the present invention will be described in detail with reference to the accompanying drawings.

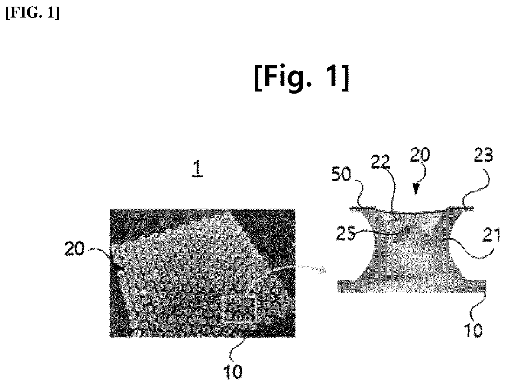

FIG. 1 is a perspective view of a skin-adhesive patch (1) capable of providing negative pressure stimulation according to an embodiment of the present invention and an enlarged partial cross-sectional view of an adhesive cup portion (20).

As shown in FIG. 1, the skin-adhesive patch (1) may include a flexible base surface portion (10) and a plurality of adhesive cup portions (20).

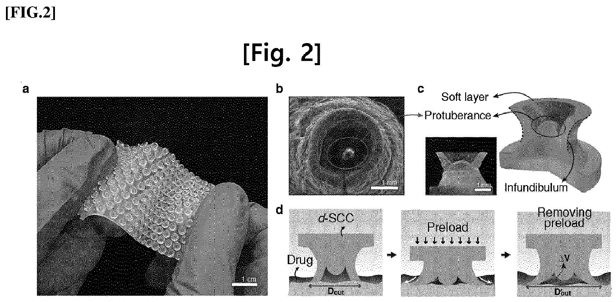

FIG. 2 includes a photograph, cross-sectional view, and design drawing of a large-area patch illustrating the structure of a protruded, octopus-inspired adhesive cup including microdome structures and curvature.

The adhesive cup structure may be a cup structure including microdomes, and may adhere to a surface through negative pressure by maximizing a volume change inside the cup.

Referring to FIG. 1(a), a photograph of the large-area patch is shown. Referring to FIGS. 1(b) to 1(c), cross-sectional views and a design drawing comparing the suction cups of an actual octopus and the adhesive cup structure may be confirmed. Referring to FIG. 1(d), the mechanism of the adhesive structure may be illustrated.

The base surface portion (10) may be formed to have flexibility.

The plurality of adhesive cup portions (20) may be formed to be arranged on a skin-adhesive surface of the base surface portion (10).

The base surface portion (10) and the adhesive cup portions (20) may be made of one or more flexible polymer materials selected from the group consisting of PDMS (Polydimethylsiloxane), Ecoflex, PU (Polyurethane), PANI (Polyaniline), and SEBS (Styrene-Ethylene/Butylene-Styrene).

The adhesive cup portion (20) may include a cup column portion (21), an adhesive flange portion (23), and a microdome portion (25).

The cup column portion (21) may have a concave column shape, wherein a side generatrix of the cup column portion (21) is formed as a concave arc in the vertical central axis direction.

A negative pressure groove (22) may be formed in a skin-contact surface of the cup column portion (21).

The adhesive flange portion (23) may be formed to extend laterally from an upper rim region of the negative pressure groove (22) of the cup column portion (21), and may be configured to enhance adhesive force by increasing the adhesive surface in contact with the skin.

The dome portion (25) may be formed to protrude upward in a raised shape from a bottom surface of the negative pressure groove (22). The dome portion (25) serves to maintain uniform distribution of internal air volume within the negative pressure groove (22). Accordingly, when the skin-adhesive patch (1) of an embodiment of the present embodiment adheres to the skin, the internal air volume capable of providing negative pressure adhesion is maintained uniformly, thereby achieving even distribution of negative pressure and improved adhesive force.

The adhesive cup portion (20) may further include a polymer precursor coating layer (50) formed on an upper surface of the adhesive flange portion (23), the polymer precursor coating layer being provided to smoothen the surface roughness and enhance adhesive force.

The outer diameter of the skin-contact surface of the adhesive cup portion (20) may range from 2 mm to 10 mm. In some cases, to effectively increase the coverage ratio of the adhesive cup portion (20), the outer diameter of the skin-contact surface may range from 2 mm to 5 mm. More preferably, the outer diameter of the adhesive cup portion (20) may range from 2 mm to 3 mm.

The polymer precursor forming the polymer precursor coating layer (50) may include at least one selected from the group consisting of PDMS (Polydimethylsiloxane), Ecoflex, PU (Polyurethane), PANI (Polyaniline), and SEBS (Styrene-Ethylene/Butylene-Styrene).

The skin-adhesive patch (1) according to an embodiment of the present invention, having the aforementioned configuration, may provide van der Waals force and negative pressure effects in dry environments, and capillary force and negative pressure effects in wet environments due to the structure of the adhesive cup portion (20).

Accordingly, when the skin-adhesive patch (1) is attached to the skin, the localized region in which the negative pressure groove (22) is formed may be maintained in a vacuum. As a result, negative pressure stimulation may be applied to the skin, thereby stimulating the stratum corneum. As a result, the delivery efficiency of various substances, including drugs, through the skin may be improved in a non-invasive manner. Furthermore, the developed skin-adhesive patch (1) may be applied to various types of drugs and utilized as a high-performance therapeutic patch by improving substance delivery efficiency through negative pressure.

A method of manufacturing a skin-adhesive patch capable of providing negative pressure stimulation to the skin according to an embodiment of the present invention may include designing a 3D mold model, fabricating a 3D mold, molding, ejecting, and forming a polymer precursor coating layer.

The designing of the 3D mold model may include designing a 3D mold model having concave shapes corresponding to a plurality of adhesive cup portions (20) for molding a skin-adhesive patch capable of providing negative pressure stimulation to the skin.

The fabricating of the 3D mold may include fabricating a 3D mold (30) for injection molding of the skin-adhesive patch (1) based on the designed 3D mold model.

The fabricating of the 3D mold may further include surface-treating the 3D mold.

The surface-treating of the mold may be carried out to enable molding of a flexible polymer material using the 3D mold as a mold base. Specifically, the surface-treating of the mold may be performed by immersing the 3D mold in a solution capable of forming a self-assembled monolayer, so that the flexible polymer material can be more easily released from the 3D mold.

The molding may include depositing a flexible polymer material onto the surface-treated 3D mold (30), curing the material, and thereby forming a skin-adhesive patch (1) having a base surface portion (10) and a plurality of adhesive cup portions (20).

The molding may be carried out through a solution-based process in which a flexible polymer material is dissolved or dispersed in an organic solvent or the like to form a solution, and is then deposited onto the 3D mold (30) by a method such as spin coating, inkjet printing, or the like.

In the molding, the flexible polymer material may include one or more selected from the group consisting of PDMS (Polydimethylsiloxane), Ecoflex, PU (Polyurethane), PANI (Polyaniline), and SEBS (Styrene-Ethylene/Butylene-Styrene).

The ejecting may include separating the molded skin-adhesive patch (1) from the 3D mold (30).

The forming of the polymer precursor coating layer may include stamping, in which a polymer precursor is applied onto the skin-adhesive surface of the skin-adhesive patch (1), and thereafter, the polymer precursor coating layer (50) and the adhesive flange portion (23) are formed by performing stamping.

Additionally, the forming of the polymer precursor coating layer may include spray coating, in which the polymer precursor coating layer (50) is formed by spraying the polymer precursor onto the skin-adhesive surface of the skin-adhesive patch (1).

The forming of the polymer precursor coating layer may further include thermally curing the polymer precursor after it has been thinly deposited by stamping or spray coating.

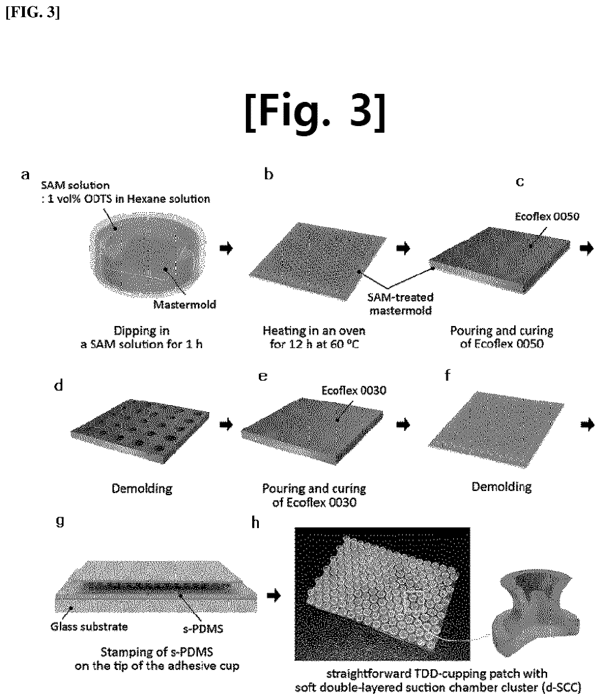

FIG. 3 is a schematic diagram illustrating a process for manufacturing an adhesive patch according to an embodiment of the present invention.

Referring to FIG. 3, manufacturing an adhesive patch according to an embodiment of the present invention may include: pretreating a master mold produced through 3D printing or the like for the molding; forming the patch through molding using a flexible polymer; and forming a soft adhesive surface via polymer precursor coating.

The polymer precursor coating layer (50) may increase the surface roughness of the adhesive flange portion (23) of the adhesive cup portion (20), thereby improving skin adhesion and minimizing skin irritation.

The adhesive flange portion (23) may also improve adhesive force by increasing the skin-contact area.

In the forming of the polymer precursor coating layer, the polymer precursor may include one or more selected from the group consisting of PDMS (Polydimethylsiloxane), Ecoflex, PU (Polyurethane), PANI (Polyaniline), and SEBS (Styrene-Ethylene/Butylene-Styrene).

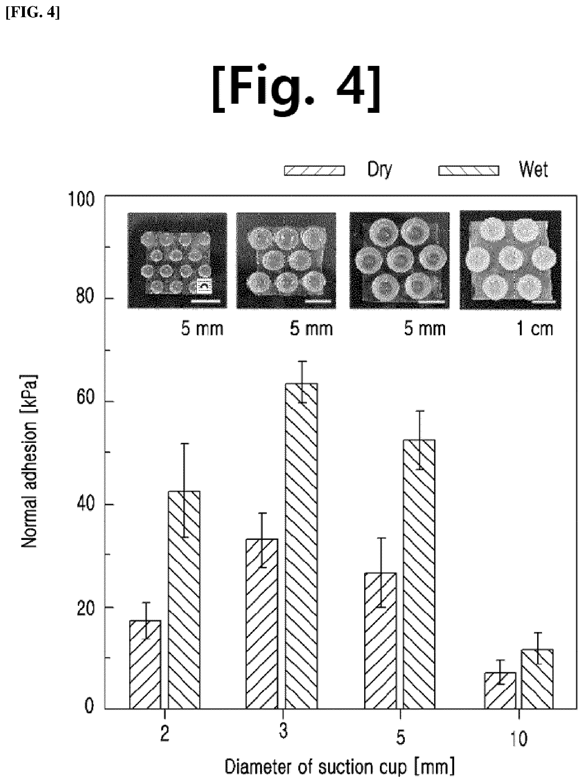

FIG. 4 is a graph showing measured vertical adhesion forces according to the scale (diameter: 2, 3, 5, and 10 mm) of the adhesive structure under dry and moist environmental conditions.

Referring to FIG. 4, it can be confirmed that the octopus-inspired adhesive structure with a diameter of 3 mm exhibits the highest vertical adhesion force when a preload of 1 N/cm² is applied.

The preload of 1 N/cm² corresponds to a pressure level that does not cause discomfort or damage to the skin.

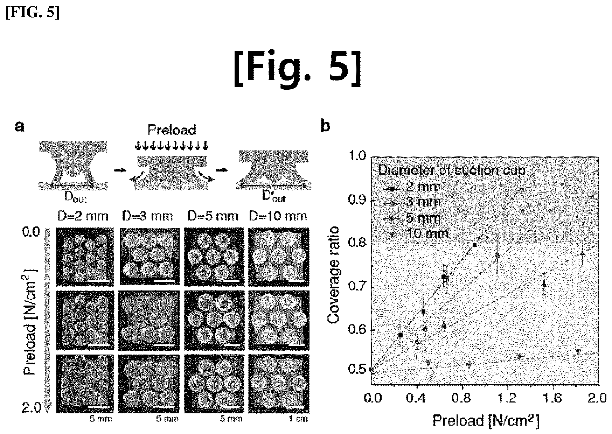

FIG. 5 illustrates (a) an image showing the increased contact area of the adhesive cup portion (20) under preload (N/cm²), depending on the outer diameter of the skin-contact surface, and (b) a graph showing the coverage ratio achieved by the adhesive cup portion (20) with respect to the outer diameter ((i) 2 mm, (ii) 3 mm, (iii) 5 mm, and (iv) 10 mm).

The term "preload" refers to the force applied to the skin-adhesive patch (1) for attachment to the skin.

Referring to FIG. 5, it can be seen that, when adhesive cup portions (20) with outer diameters of (i) 2 mm, (ii) 3 mm, (iii) 5 mm, and (iv) 10 mm are applied to the skin and preload is applied, the contact area of the adhesive cup portion (20) increases.

The coverage ratio of the adhesive cup portion (20) was found to increase as the preload increased. In addition, it was confirmed that the smaller the outer diameter of the skin-contact surface of the adhesive cup portion (20), the greater the resulting coverage ratio. Accordingly, the outer diameter of the skin-contact surface of the adhesive cup portion (20) may range from 2 mm to 10 mm.

For effective enhancement of the coverage ratio, the outer diameter may range from 2 mm to 5 mm.

More preferably, the outer diameter of the adhesive cup portion (20) may range from 2 mm to 3 mm.

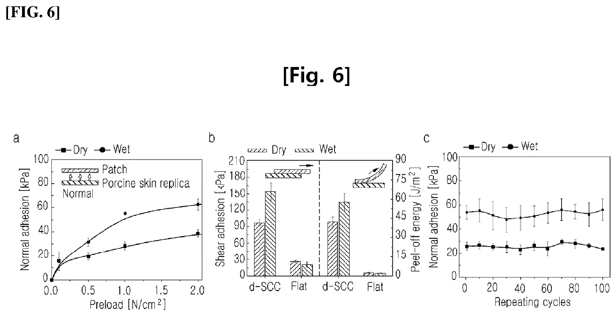

FIG. 6 illustrates the adhesion performance of the patch on a pig skin replica, including (a) vertical adhesion performance depending on preload, (b) horizontal and peeling adhesion performance of the octopus-inspired suction cup structure (d-SCC) and a flat (non-structured) patch, and (c) adhesion performance of the d-SCC structure after 100 repeated attachment/detachment cycles.

Referring to FIG. 6(a), it was confirmed that, in both wet and dry conditions, the vertical adhesion performance increased with increasing preload applied to attach the skin-adhesive patch (1) to the skin. Additionally, across the entire preload range, it was confirmed that the vertical adhesion performance was higher under wet conditions than under dry conditions.

The "dry" condition refers to a normal skin surface without any liquid, while the "wet" condition refers to a skin surface sufficiently wetted with liquid.

Typically, conventional adhesives exhibit reduced adhesion, performance in the presence of moisture or liquids, as such conditions lower the surface energy of the bonding interface. However, the adhesive cup structure according to an embodiment of the present invention is characterized in that it enhances adhesion on wet skin surfaces due to sealing of the suction structure and capillary forces, thereby providing improved performance under wet conditions.

Generally, conventional adhesives exhibit reduced adhesion in the presence of moisture or liquids, as such substances decrease the surface energy of the adhesion interface. However, the adhesive cup according to an embodiment of the present invention is characterized by enhanced adhesion on wet skin surfaces, owing to sealing within the suction section and the action of capillary forces.

Referring to FIG. 6(b), it was confirmed that the octopus-inspired suction cup structure (d-SCC) according to an embodiment of the present invention exhibited significantly superior performance in both horizontal and peeling adhesion compared to a flat (non-structured) patch. In addition, the octopus-inspired suction cup structure (d-SCC) according to an embodiment of the present invention showed better performance under wet conditions than under dry conditions.

Referring to FIG. 6(c), it was confirmed that the octopus-inspired suction cup structure (d-SCC) according to an embodiment of the present invention exhibited superior adhesion performance under wet conditions compared to dry conditions across the entire range, even after 100 repeated attachment and detachment cycles.

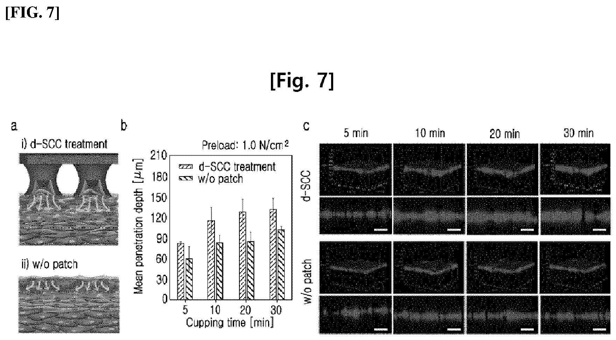

FIG. 7 includes: (a) schematic diagrams comparing the cases with and without application of the d-SCC; (b) measurement data of fluorescence particle (rhodamine B) delivery depth on pig skin according to the application time of the d-SCC (5, 10, 20, and 30 minutes); and (c) images showing the delivery of fluorescence particle (rhodamine B) into the skin according to the application time of the d-SCC (5, 10, 20, and 30 minutes).

Referring to FIG. 7, it was confirmed that when d-SCC was applied for 30 minutes, the delivery depth of the fluorescent particles (rhodamine B) reached approximately 130 µm, which was the greatest among the tested conditions.

In addition, it was confirmed that when the d-SCC was applied for 30 minutes, the delivery depth of the fluorescent particles (rhodamine B) improved by up to 44% compared to the case in which the fluorescent particles were applied without a patch (w/o patch).

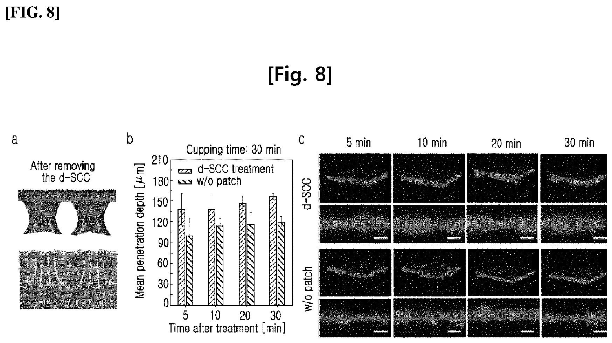

FIG. 8 includes: (a) a schematic diagram illustrating the condition after removal of the d-SCC; (b) measurement data showing the delivery depth of fluorescent particles (rhodamine B) on pig skin according to the elapsed time (5, 10, 20, and 30 minutes) after the d-SCC was applied for 30 minutes and then removed; and (c) images showing the delivery of fluorescent particles (rhodamine B) corresponding to each elapsed time (5, 10, 20, and 30 minutes) after removal of the d-SCC following 30 minutes of application.

Referring to FIG. 8, it was confirmed that, 30 minutes after removal of the d-SCC following 30 minutes of application, the delivery depth of the fluorescent particles (rhodamine B) reached over 150 µm, which was the greatest among the tested conditions.

In addition, it was confirmed that, 30 minutes after removal of the d-SCC following 30 minutes of application, the delivery depth of the fluorescent particles (rhodamine B) improved by up to 36% compared to the case in which the fluorescent particles were applied without a patch (w/o patch).

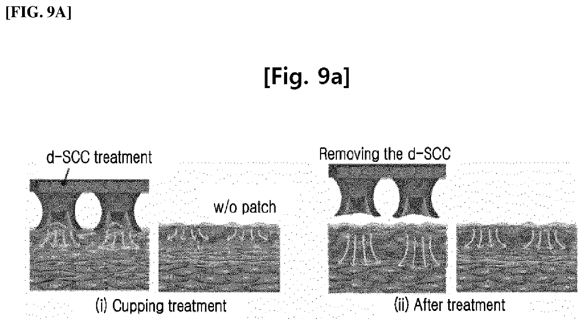

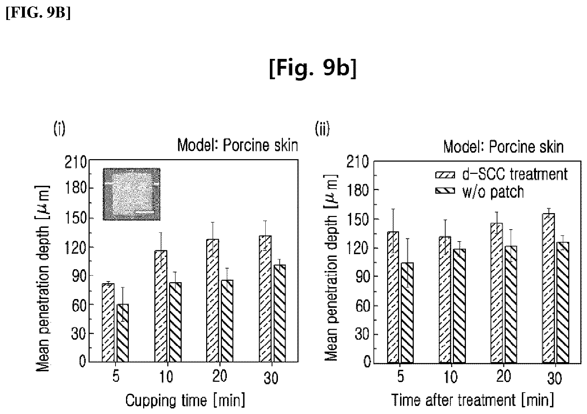

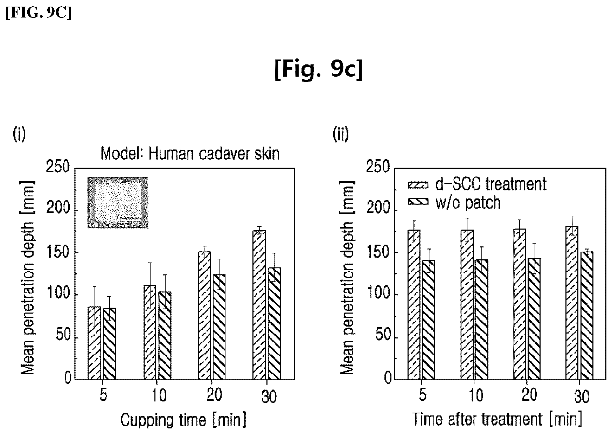

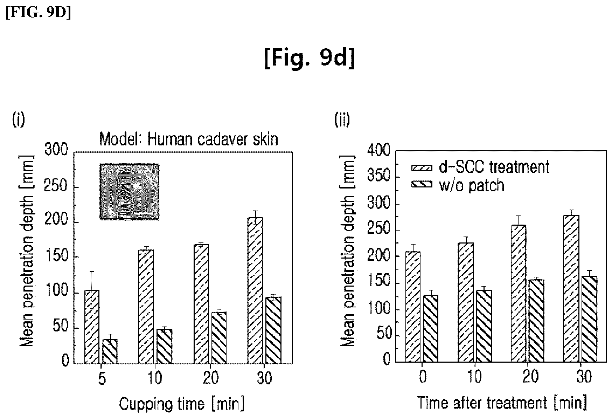

FIG. 9 is a graph showing the measured delivery depth of fluorescent particles enhanced by the d-SCC on various types of skin, including pig skin, human skin, and artificial skin.

Referring to FIG. 9(b), in the case of pig skin, it was confirmed that the delivery depth of fluorescent particles (rhodamine B) improved by up to 44% compared to the case in which the fluorescent particles were applied without a patch (w/o patch) after 30 minutes of patch application according to an embodiment of the present invention. Furthermore, 30 minutes after removal of the patch, the delivery depth was still improved by up to 36% compared to the w/o patch case.

In the case of human skin, it was confirmed that the delivery depth of fluorescent particles (rhodamine B) improved by up to 56% after 30 minutes of patch application, compared to the case in which the fluorescent particles were applied without a patch (w/o patch).

Furthermore, it was confirmed that 30 minutes after removal of the patch according to an embodiment of the present invention, the delivery depth of fluorescent particles (rhodamine B) improved by up to 31% compared to the case in which the fluorescent particles were applied without a patch (w/o patch).

In the case of artificial skin, it was confirmed that the delivery depth of fluorescent particles (rhodamine B) improved by up to 138% after 30 minutes of patch application according to an embodiment of the present invention, compared to the case in which the fluorescent particles were applied without a patch (w/o patch).

Furthermore, it was confirmed that 30 minutes after removal of the patch according to an embodiment of the present invention, the delivery depth of fluorescent particles (rhodamine B) improved by up to 95% compared to the case in which the fluorescent particles were applied without a patch (w/o patch).

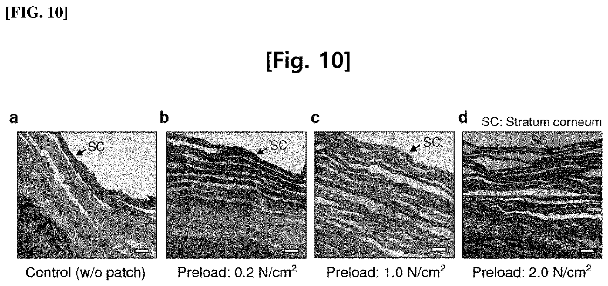

FIG. 10 shows images illustrating the degree of stratum corneum deformation in (pig) skin under different preload conditions.

Referring to FIG. 10, it was confirmed that the distance between layers of the stratum corneum increased when preload was applied and the patch was attached, indicating deformation. In addition, as the preload increased, greater negative pressure was transmitted to the skin, resulting in a more significant degree of stratum corneum deformation. These results suggest that application of the patch may have a meaningful effect on the stratum corneum of the skin and enhance the efficiency of transdermal substance delivery.

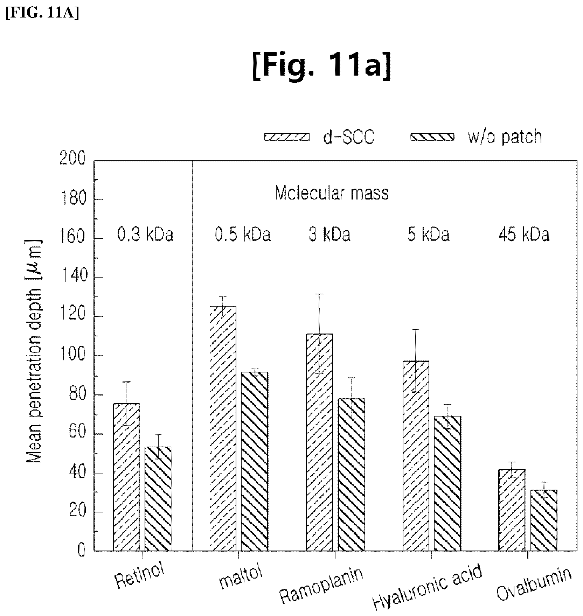

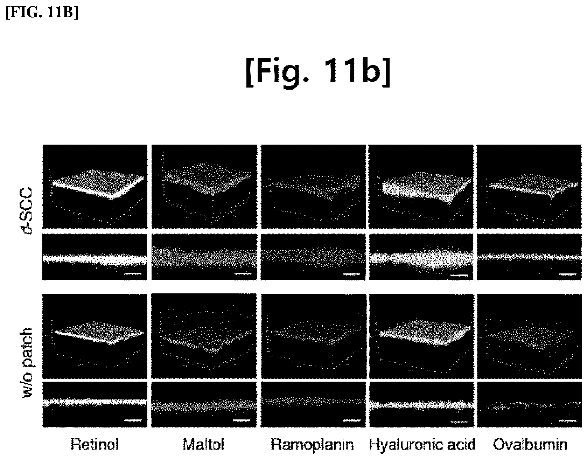

FIG. 11 includes: (a) a graph showing the improvement in delivery depth by the d-SCC for various drugs with different molecular weights on pig skin; and (b) images showing the delivered states of the respective drugs under each condition.

Referring to FIG. 11(a), it was confirmed that the octopus-inspired suction cup structure (d-SCC) according to an embodiment of the present invention, when applied together with the drug Maltol, resulted in the greatest delivery depth among the tested conditions.

It was also confirmed that the octopus-inspired suction cup structure (d-SCC) according to an embodiment of the present invention exhibited excellent delivery depth even for substances with various solubilities, when applied in combination with the lipophilic compound Retinol and hydrophilic compounds such as Maltol, Ramoplanin, Hyaluronic acid, and Ovalbumin.

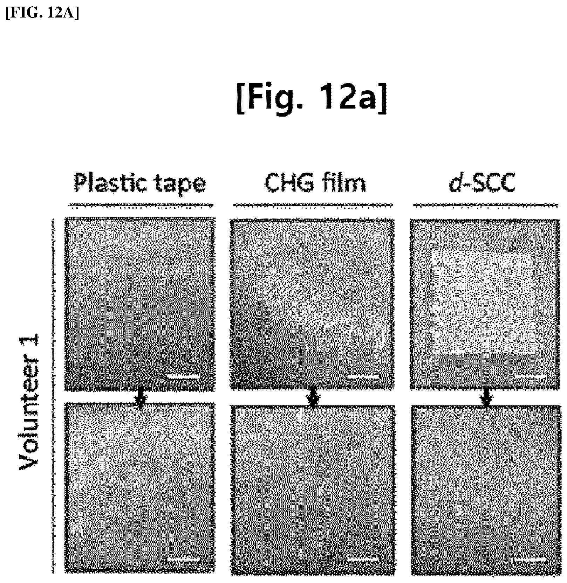

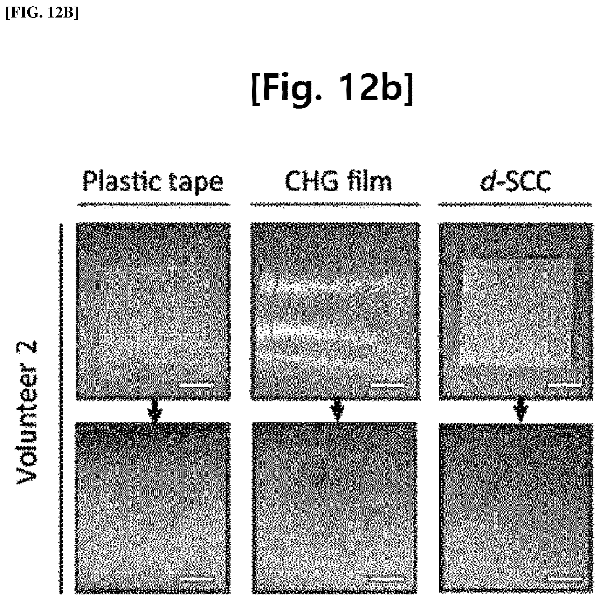

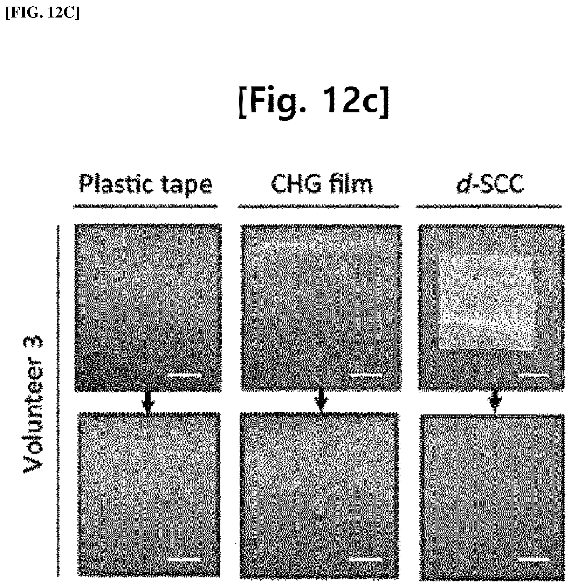

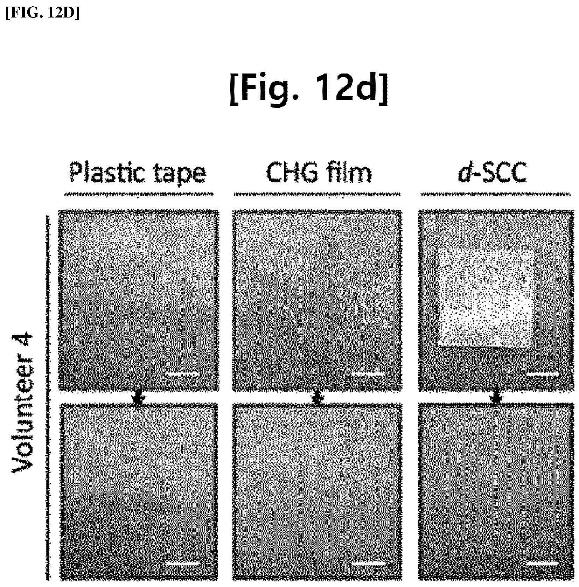

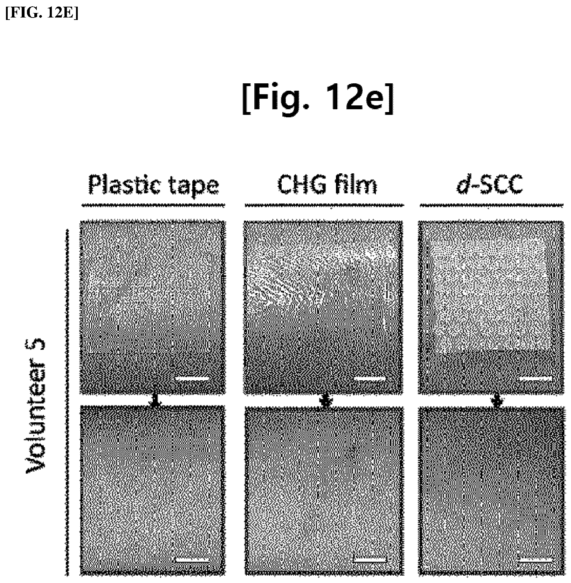

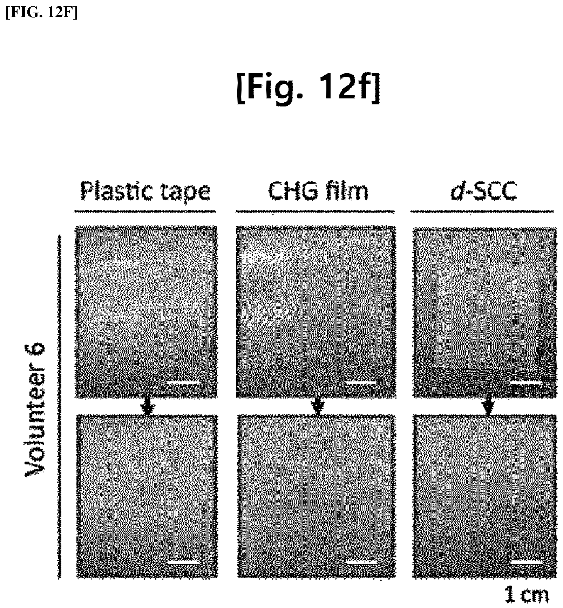

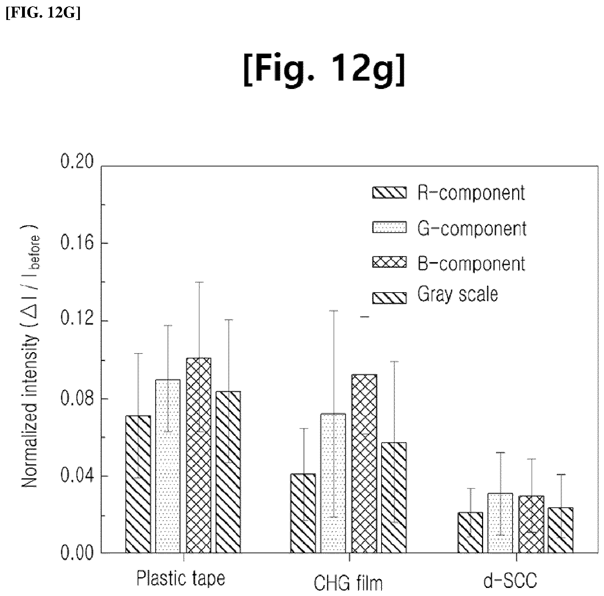

FIG. 12 includes: (a) images showing the application of commercial products (surgical plastic tape and chlorhexidine gluconate film) and the d-SCC patch according to an embodiment of the present invention to various areas of human skin; and (b) a graph comparing the degree of skin irritation caused by the commercial products and the d-SCC patch.

Referring to FIG. 12, redness caused by irritation was evaluated based on RGB intensity values measured one minute after removal of each patch, including various commercial products and the d-SCC patch according to an embodiment of the present invention. It was confirmed that the d-SCC patch according to an embodiment of the present invention showed the smallest change in RGB values compared to other commercial products such as surgical plastic tape and chlorhexidine gluconate film. This indicates that the d-SCC patch according to an embodiment of the present invention caused the least skin irritation compared to other commercial products such as the surgical plastic tape or chlorhexidine gluconate film.

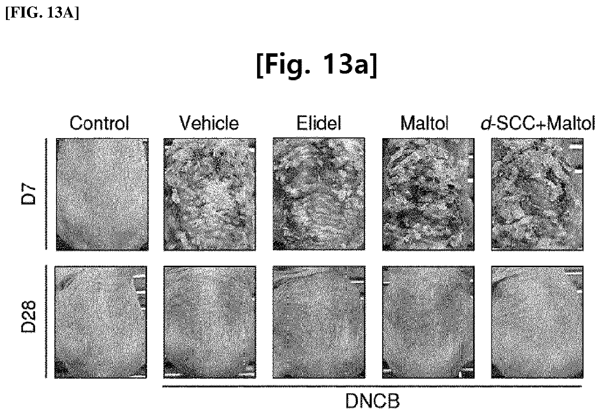

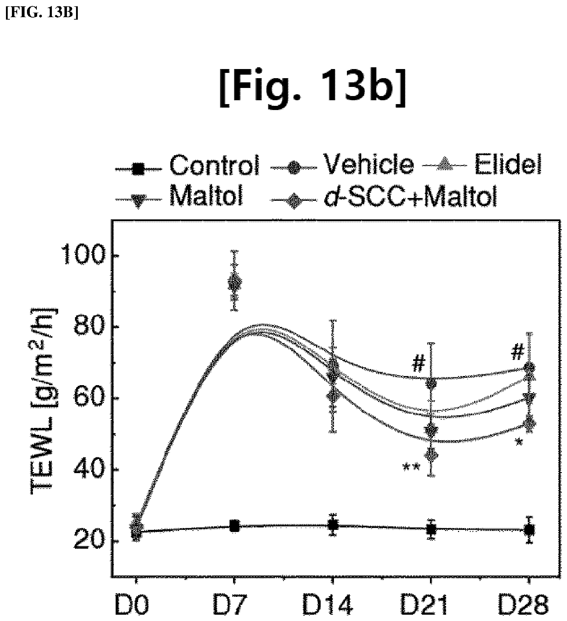

FIG. 13 includes:(a) images showing the skin recovery progression in atopic dermatitis-induced mice following the combined application of various drugs and the d-SCC; and (b) a graph illustrating transepidermal water loss over time resulting from the combined application of the respective drugs and the d-SCC.

Referring to FIG. 13, it was confirmed that when the atopic dermatitis treatment drug maltol was applied in combination with the d-SCC patch according to an embodiment of the present invention, the transepidermal water loss (TEWL) of the skin recovered to a level closest to that of normal (control) skin.

The foregoing description of the present invention is intended for illustrative purposes only, and it will be understood by those skilled in the art that various modifications and changes may be made without departing from the spirit or essential characteristics of the invention. [The preferred ranges are provided to illustrate exemplary embodiments, and do not limit the scope of the present invention unless expressly recited in the claims.] Therefore, the embodiments described above should be regarded as illustrative in all respects and not as restrictive. For example, components described as being implemented in a singular form may be implemented in a distributed manner, and likewise, components described as being distributed may be implemented in a combined form.

The scope of the present invention is defined by the appended claims, and all modifications or equivalent arrangements that fall within the meaning and scope of the claims are to be interpreted as being included in the scope of the invention.

### [DESCRIPTION OF REFERENCE NUMERALS]

1: Skin adhesive patch
10: Base surface portion
20: Adhesive cup portion
21: Cup column portion
22: Negative pressure groove
23: Adhesive flange portion
25: Dome portion
30: 3D mold
40: Flexible polymer material
50: Polymer precursor coating layer

## Claims

1. A skin adhesive patch capable of providing negative pressure stimulation, comprising:
a base surface portion (10) having flexibility; and
a plurality of adhesive cup portions (20) arranged in plurality on a skin adhesive surface of the base surface portion (10).

2. The skin adhesive patch capable of providing negative pressure stimulation according to claim 1,
wherein the base surface portion (10) and the adhesive cup portions (20) include one or more flexible polymer materials selected from the group consisting of PDMS (Polydimethylsiloxane), Ecoflecx, PU (Polyurethane), PANI (Polyaniline), and SEBS (Styrene-Ethylene/Butylene-Styrene).

3. The skin adhesive patch capable of providing negative pressure stimulation according to claim 1,
wherein each of the adhesive cup portions (20) comprises:
a cup column portion (21) having a negative pressure groove (22) formed on a skin adhesive surface;
an adhesive flange portion (23) extending laterally from the cup column portion (21) in the upper edge region of the negative pressure groove (22); and
a dome portion (25) protruding convexly from a bottom surface of the negative pressure groove (22).

4. The skin adhesive patch capable of providing negative pressure stimulation according to claim 3,
wherein the cup column portion (21) has a concave pillar shape, with its lateral generatrix curved inward in an arc shape along the vertical central axis of the cup column portion (21).

5. The skin adhesive patch capable of providing negative pressure stimulation according to claim 3,
wherein each of the adhesive cup portions (20) further comprises a polymer precursor coating layer (50) formed on an upper surface of the adhesive flange portion (23) to smooth the surface roughness and enhance adhesion performance.

6. The skin adhesive patch capable of providing negative pressure stimulation according to claim 5,
wherein the polymer precursor forming the polymer precursor coating layer (50) comprises at least one selected from the group consisting of PDMS (Polydimethylsiloxane), Ecoflex, PU (Polyurethane), PANI (Polyaniline), and SEBS (Styrene-Ethylene/Butylene-Styrene).

7. A method of manufacturing a skin adhesive patch (1) capable of providing negative pressure stimulation according to claim 1, the method comprising:
designing a 3D mold model having concave shapes corresponding to the plurality of adhesive cup portions (20);
fabricating a 3D mold (30) based on the designed 3D mold model;
molding a skin adhesive patch (1) including a base surface portion (10) and a plurality of adhesive cup portions (20) by depositing a flexible polymer material in the fabricated 3D mold (30) and curing the material;
separating the molded skin adhesive patch (1) from the 3D mold (30); and
forming a polymer precursor coating layer (50) on a skin adhesive surface of the skin adhesive patch (1).

8. The method for manufacturing a skin adhesive patch capable of providing negative pressure stimulation according to claim 7,
wherein the fabricating of the 3D mold further comprises surface treating the 3D mold.

9. The method for manufacturing a skin adhesive patch capable of providing negative pressure stimulation according to claim 7,
wherein the molding is performed through a solution process using the flexible polymer material.

10. The method for manufacturing a skin adhesive patch capable of providing negative pressure stimulation according to claim 7,
wherein the flexible polymer material used in the molding comprises at least one selected from the group consisting of PDMS (Polydimethylsiloxane), Ecoflex, PU (Polyurethane), PANI (Polyaniline), and SEBS (Styrene-Ethylene/Butylene-Styrene).

11. The method for manufacturing a skin adhesive patch capable of providing negative pressure stimulation according to claim 7,
wherein the forming of the polymer precursor coating layer (50) comprises stamping in which the polymer precursor coating layer (50) and the adhesive flange portion (23) are formed by stamping after laminating a polymer precursor is laminated onto a skin adhesive surface of the skin adhesive patch (1).

12. The method for manufacturing a skin adhesive patch capable of providing negative pressure stimulation according to claim 7,
wherein the forming of the polymer precursor coating layer comprises spray coating in which the polymer precursor coating layer (50) is formed by spraying a polymer precursor onto a skin adhesive surface of the skin adhesive patch (1).

13. The method for manufacturing a skin adhesive patch capable of providing negative pressure stimulation according to claim 7,
wherein the polymer precursor used in forming the polymer precursor coating layer comprises at least one selected from the group consisting of PDMS (Polydimethylsiloxane), Ecoflex, PU (Polyurethane), PANI (Polyaniline), and SEBS (Styrene-Ethylene/Butylene-Styrene).
